# FASCICULE DE BREVET EUROPEEN

(11) **EP 2 624 912 B1**
(45) Date de publication et mention de la délivrance du brevet: **25.04.2018**
(21) Numéro de dépôt: 10782654.7
(22) Date de dépôt: 08.10.2010
(51) Int. Cl.: A61N 5/06

(54) **APPAREIL DE STIMULATION LOCALE D'UN SUJET**
VORRICHTUNG ZUR LOKALEN STIMULIERUNG EINER PERSON
APPARATUS FOR LOCALLY STIMULATING A SUBJECT

(43) Date de publication de la demande: 14.08.2013
(73) Titulaire: Luxomed, 59120 Loos (FR)
(72) Inventeur: GABERT, André, B-7500 Tournai (BE)
(74) Mandataire: Balesta, Pierre
(86) Numéro de dépôt international: PCT/FR2010/052120
(87) Numéro de publication internationale: WO 2012/045918

(56) Documents cités:
- EP-A1- 1 426 026
- EP-A1- 1 645 262
- WO-A1-2010/138025
- DE-A1- 19 737 675
- GB-A- 1 350 684
- US-A- 5 195 517
- US-A1- 2005 203 592

## Description

La présente invention a pour objet un appareil de stimulation locale d'un sujet. Elle trouve notamment son application à la stimulation de points tels que des points d'acupuncture, d'auriculothérapie ou de réflexothérapie, appelés points réflexes, ou à la stimulation de terminaisons nerveuses, par un faisceau infrarouge.

On connaît l'acupuncture comme l'une des composantes de la médecine traditionnelle chinoise notamment, et dont la caractéristique la plus représentative consiste dans le traitement par implantation d'aiguilles.

Plus généralement, l'acupuncture est pratique médicale chinoise ancestrale qui repose sur l'idée qu'il existe un flux d'énergie circulant sous la peau selon des chemins privilégiés et véhiculant deux types d'énergie, l'une positive et l'autre négative. Selon ce principe, les maladies proviennent d'une mauvaise circulation, empêchée ou déséquilibrée, de ces énergies. L'acupuncture est donc considérée, selon ce principe, comme une des méthodes pour rétablir la bonne circulation du flux d'énergie, au moyen d'aiguilles plantées sur des chemins privilégiés tels qu'expliqué plus haut, en des points particuliers susceptibles d'agir sur un organe donné.

Par ailleurs, on connaît l'auriculothérapie, ou acupuncture auriculaire, comme une pratique traditionnelle qui repose sur l'hypothèse selon laquelle il existe une correspondance entre l'oreille externe et les différents organes du corps. Selon ce principe, il est possible dans certains cas d'agir sur certaines organes en piquant le pavillon de l'oreille à l'aide d'aiguilles stériles.

Selon ces deux méthodes traditionnelles, on stimule donc localement un sujet en vue d'obtenir un effet particulier.

Des alternatives à l'utilisation d'aiguilles ont été envisagées, dans lesquelles l'aiguille est remplacée par un faisceau infrarouge.

On connaît par exemple des dispositifs destinés à impartir une stimulation locale à un sujet au moyen d'un faisceau laser. Néanmoins, l'utilisation et le contrôle d'un tel faisceau laser pose problème, avec notamment des risques non négligeables de brûlure, et la nécessité d'une grande précision dans l'application du faisceau qui rend ces dispositifs difficiles à utiliser.

On connaît par ailleurs des appareils à lampe à infrarouge, par exemple pour le traitement de la lombalgie, qui sont moins dangereux et moins difficiles à manipuler que les dispositifs mentionnés précédemment du fait des propriété de l'infrarouge, mais qui ne font que stimuler par la chaleur des zones larges et superficielles, sans concentrer un faisceau en sorte de permettre la stimulation d'un point précis d'acupuncture.

Enfin, on connaît du document FR 2 371 935 et du document DE 197 37 675 un dispositif permettant d'appliquer un faisceau infrarouge localement.

Toutefois, un tel dispositif ne fonctionne que sur la base d'une gamme de fréquences de découpes élevées, précisément supérieures à 70 Hz et allant jusqu'à plusieurs milliers d'hertz (de l'ordre de 5000 Hz). Par fréquence de découpe, on entend la fréquence des impulsions électriques excitatrices de la source infrarouge.

En outre, dans un tel dispositif, la longueur d'onde utilisée est sans limite dans le domaine infrarouge (supérieur à environ 700 nm).

Or, l'effet recherché, à savoir notamment la stimulation locale d'un point réflexes ou d'une terminaison nerveuse, n'est pas obtenu avec des fréquences de découpe élevées, ni à des longueurs d'onde infrarouge élevées.

Un des problèmes qui se pose alors est donc de disposer d'un appareil qui permettent une stimulation locale d'un sujet, notamment au niveau de points d'acupuncture ou d'auriculothérapie, par l'intermédiaire d'un faisceau infrarouge.

Par ailleurs, les dispositifs connus sont peu pratiques lorsqu'il s'agit de permettre la stimulation locale selon un protocole prédéterminé qui peut être complexe en termes de localisation des zones à stimuler et de paramètres du faisceau infrarouge à appliquer sur chacune de ces zones.

L'utilisateur doit bien connaître la localisation de ces zones et les paramètres associés pour éviter toute erreur de protocole.

A titre d'exemple, pour des protocoles dans lesquels la durée de la stimulation varie pour chaque point, sur une séquence de points relativement importante, il est difficile de mémoriser à la fois la localisation de tous ces points et toutes les durées d'application successives.

Il est difficile pour l'utilisateur d'interrompre le déroulement du protocole pour consulter les données de celui-ci en cas d'oubli, par exemple dans un livre.

L'objet de l'invention est donc d'apporter une solution aux problèmes précités parmi d'autres problèmes. L'invention est définie par les revendications. L'invention se rapporte donc, selon un premier aspect, à un appareil destiné à appliquer une stimulation locale sur un point d'un sujet, notamment un point d'acupuncture, d'auriculothérapie ou de réflexothérapie, dit point réflexe, ou sur une terminaison nerveuse.

Dans toute la suite de la description, on utilise indifféremment l'expression point réflexe pour désigner un point à stimuler tel un point d'acupuncture, d'auriculothérapie ou de réflexothérapie, et une terminaison nerveuse à stimuler.

L'appareil comprend une unité de commande qui contrôle une source infrarouge pour l'émission d'un faisceau infrarouge.

Il comprend également une unité de stockage, un moyen d'application du faisceau infrarouge sur le point réflexe, et une interface utilisateur.

Cette interface utilisateur permet notamment à l'utilisateur de déclencher l'émission d'au moins un faisceau infrarouge.

De façon caractéristique, l'unité de stockage est apte à stocker au moins une séquence de valeurs d'un ou plusieurs paramètres d'application du faisceau infrarouge, notamment la durée d'application et/ou la puissance optique restituée, correspondant à un protocole de soin, par exemple dans le cadre d'un programme de perte de poids ou de relaxation.

Par ailleurs, l'interface utilisateur comprend des premiers moyens de sélection de la séquence de valeurs.

Enfin, l'unité de commande est apte à contrôler le ou les paramètres et à commander l'émission de plusieurs faisceaux infrarouge successifs selon la séquence de valeurs, en sorte de permettre l'application sur une séquence de points par l'intermédiaire du moyen d'application, d'une séquence de faisceaux infrarouge correspondant à la séquence de valeurs.

Ainsi, l'utilisateur bénéficie d'une assistance dans l'application d'un protocole de stimulation basé sur une séquence précise de valeurs de paramètres du faisceau infrarouge, dont la durée d'application de chaque faisceau, cette séquence étant enregistrée au préalable dans l'appareil.

Dans une première variante de réalisation, l'unité de stockage est apte à stocker les positions respectives des points de la séquence sur lesquels les faisceaux infrarouge successifs doivent être respectivement appliqués, et l'interface utilisateur comprend des moyens d'indication de ces positions Les moyens d'indication comprennent une représentation graphique d'au moins une partie du sujet sur laquelle sont positionnés les points de la séquence.

Ainsi, l'utilisateur bénéficie là encore d'une assistance dans l'application du protocole de stimulation basé également sur une séquence précise de points à stimuler dont la localisation lui est indiquée par l'appareil.

Dans une autre variante de réalisation, éventuellement en combinaison avec la précédente, l'interface utilisateur comprend des deuxièmes moyens de sélection d'au moins un mode d'application manuel et/ou d'au moins un mode d'application automatique.

Dans le mode d'application manuel, l'émission de chaque faisceau infrarouge de la séquence de faisceaux infrarouge est déclenchée manuellement.

Dans le mode d'application automatique, l'émission du premier faisceau infrarouge de la séquence de faisceaux lumineux est déclenchée manuellement, et l'émission de chaque faisceau infrarouge suivant dans la séquence de faisceaux infrarouge est déclenchée automatiquement après un laps de temps prédéterminé à partir de la fin de l'émission du précédent faisceau infrarouge dans cette séquence.

De préférence, le laps de temps prédéterminé entre la fin de l'émission d'un faisceau infrarouge et le début de l'émission du faisceau infrarouge suivant est constant sur toute la séquence de faisceau infrarouge.

Ce laps de temps prédéterminé peut alors être stocké dans l'unité de stockage.

Alternativement, le laps de temps prédéterminé entre la fin de l'émission d'un faisceau lumineux et le début de l'émission du faisceau lumineux suivant varie sur la séquence de faisceau lumineux.

Les différents laps de temps prédéterminés peuvent alors être stockés dans l'unité de stockage.

Dans encore une autre variante de réalisation, éventuellement en combinaison avec une ou plusieurs quelconques des précédentes, l'interface utilisateur comprend des troisièmes moyens de sélection permettant de sélectionner un mode expert.

Dans ce mode expert, l'interface utilisateur permet la saisie et l'enregistrement dans l'unité de stockage des positions des points d'une séquence de points, et/ou la saisie et l'enregistrement dans cette unité de stockage d'une séquence correspondante de valeurs du ou des paramètres d'application du faisceau infrarouge.

Dans encore une autre variante de réalisation, éventuellement en combinaison avec une ou plusieurs quelconques des précédentes, l'appareil comprend un moyen de déclenchement manuel de l'émission d'un faisceau infrarouge.

De préférence, ce moyen de déclenchement manuel, par exemple de type bouton de commande, est disposé sur le moyen d'application.

L'appareil peut aussi comprendre un ou plusieurs témoins lumineux de fonctionnement du moyen d'application, ce témoin lumineux étant activé par activation du moyen de déclenchement.

Dans ce cas, le moyen de déclenchement manuel peut être translucide, le témoin lumineux étant alors une source de lumière, par exemple de type LED, disposée à l'intérieur du moyen d'application en regard du moyen de déclenchement.

Alternativement, le témoin lumineux étant une source de lumière, par exemple de type LED, il peut être disposé à l'intérieur du moyen d'application en regard d'un élément annulaire translucide présent sur le pourtour du moyen d'application.

Dans encore une autre variante de réalisation, éventuellement en combinaison avec une ou plusieurs quelconques des précédentes, l'interface utilisateur comprend des quatrièmes moyens de sélection d'au moins un mode d'application par impulsion et/ou au moins un mode d'application continu.

Dans le mode d'application par impulsion, l'unité de commande permet l'émission d'un faisceau infrarouge en délivrant pendant un temps déterminé un train d'impulsions.

Dans le mode d'application continu, l'unité de commande permet l'émission d'un faisceau infrarouge en délivrant une seule impulsion de durée déterminée.

De préférence, l'interface utilisateur permet de régler le temps d'application du train d'impulsions dans le mode par impulsion, et/ou la durée de l'impulsion dans le mode continu.

Dans une autre variante de réalisation, éventuellement en combinaison avec une ou plusieurs quelconques des précédentes, l'appareil permet, en fonctionnement, l'émission d'un faisceau infrarouge divergeant et présentant un angle d'ouverture sensiblement égal à 100°, de sorte que lorsque le moyen d'application se trouve en contact direct avec la surface du sujet, la zone stimulée par application du faisceau lumineux, à une profondeur sous la surface du sujet sensiblement comprise entre 0 cm et 6 cm, est sensiblement circulaire et présente un diamètre sensiblement compris entre 0,7 cm et 3 cm.

Dans encore une autre variante de réalisation, éventuellement en combinaison avec une ou plusieurs quelconques des précédentes, la source infrarouge comprend plusieurs émetteurs infrarouge, dont au moins une LED infrarouge.

Dans encore une autre variante de réalisation, éventuellement en combinaison avec une ou plusieurs quelconques des précédentes, l'unité de commande permet de délivrer une ou plusieurs impulsions de forme variable, par exemple sensiblement sinusoïdale ou carrée, de durée variable, et de fréquence égale à la fréquence de découpe, à une interface de puissance qui transforme ces impulsions en courant d'alimentation de la source infrarouge. Un procédé d'application d'une stimulation locale sur un point d'un sujet est également décrit. Le procédé consiste notamment en l'utilisation d'un moyen d'application d'un faisceau infrarouge émis par une source infrarouge contrôlée par une unité de commande.

De façon caractéristique, on applique une séquence de faisceaux infrarouge respectivement sur une séquence de points de ce sujet.

L'application de chaque faisceau infrarouge est paramétrée par un ou plusieurs paramètres, notamment sa durée d'application et/ou sa puissance optique restituée selon une séquence prédéterminée de valeurs du ou des paramètres.

Dans une première variante de mise en oeuvre, le procédé consiste en outre à indiquer à l'utilisateur la position des points de la séquence de points sur lesquels les faisceaux infrarouge successifs doivent être respectivement appliqués.

De préférence, l'indication de la position de ces points est donnée par l'intermédiaire d'une représentation graphique d'au moins une partie du sujet dans laquelle sont positionnés les points de la séquence.

Par ailleurs, peuvent aussi être affichés le nom de la séquence de points, correspondant à un type de soin particulier, les paramètres du faisceau infrarouge, et d'autres paramètres tels que des paramètres correspondant au mode de stimulation (par exemple normal ou intensif).

Dans une deuxième variante de mise en oeuvre, éventuellement en combinaison avec la précédente, l'émission du premier faisceau infrarouge de la séquence de faisceaux infrarouge est déclenchée manuellement, et l'émission de chaque faisceau infrarouge suivant dans la séquence de faisceaux infrarouge est déclenchée automatiquement ou manuellement.

De préférence, lorsque l'émission de chaque faisceau infrarouge suivant le premier faisceau infrarouge de la séquence est déclenchée automatiquement, ce déclenchement intervient après un laps de temps prédéterminé à partir de la fin de l'émission du précédent faisceau infrarouge dans cette séquence.

Ce laps de temps prédéterminé entre la fin de l'émission d'un faisceau infrarouge et le début de l'émission du faisceau infrarouge suivant peut alors être constant sur toute la séquence de faisceau infrarouge.

Alternativement, ce laps de temps prédéterminé entre la fin de l'émission d'un faisceau infrarouge et le début de l'émission du faisceau infrarouge suivant peut varier sur la séquence de faisceau infrarouge.

Dans une autre variante de mise en oeuvre, éventuellement en combinaison avec une ou plusieurs quelconques des précédentes, pour l'émission d'un faisceau infrarouge, l'unité de commande délivre pendant un temps déterminé un train d'impulsions, ou délivre une seule impulsion de durée déterminée.

Dans une autre variante de mise en oeuvre, éventuellement en combinaison avec une ou plusieurs quelconques des précédentes, le ou les faisceaux infrarouge sont divergents et présentent un angle d'ouverture sensiblement égal à 100°, de sorte que lorsque le moyen d'application se trouve en contact direct avec la surface du sujet, la zone stimulée par application du ou des faisceaux lumineux, à une profondeur sous la surface du sujet sensiblement comprise entre 0 cm et 6 cm, est sensiblement circulaire et présente un diamètre sensiblement compris entre 0,7 cm et 3 cm.

Dans une autre variante de mise en oeuvre, éventuellement en combinaison avec une ou plusieurs quelconques des précédentes, l'unité de commande délivre une ou plusieurs impulsions de forme variable, par exemple sensiblement sinusoïdale ou carrées, de durée variable, et de fréquence égaie à la fréquence de découpe, à une interface de puissance qui transforme ces impulsions en courant d'alimentation de la source infrarouge.

D'autres caractéristiques et avantages de l'invention apparaîtront plus clairement et de manière complète à la lecture de la description ci-après des variantes préférées, lesquelles sont données à titre d'exemples non limitatifs et en référence aux dessins annexés suivants.
- figure 1 : représente schématiquement et fonctionnellement un exemple d'appareil selon l'invention,
- figure 2 : représente schématiquement l'application avec l'appareil selon l'invention d'un faisceau lumineux infrarouge pour la stimulation locale d'un sujet,
- figure 3 : représente schématiquement un exemple de moyen d'application du faisceau infrarouge de l'appareil selon l'invention,
- figures 4a, 4b : représentent schématiquement et respectivement deux états de l'interface utilisateur de l'appareil selon l'invention.

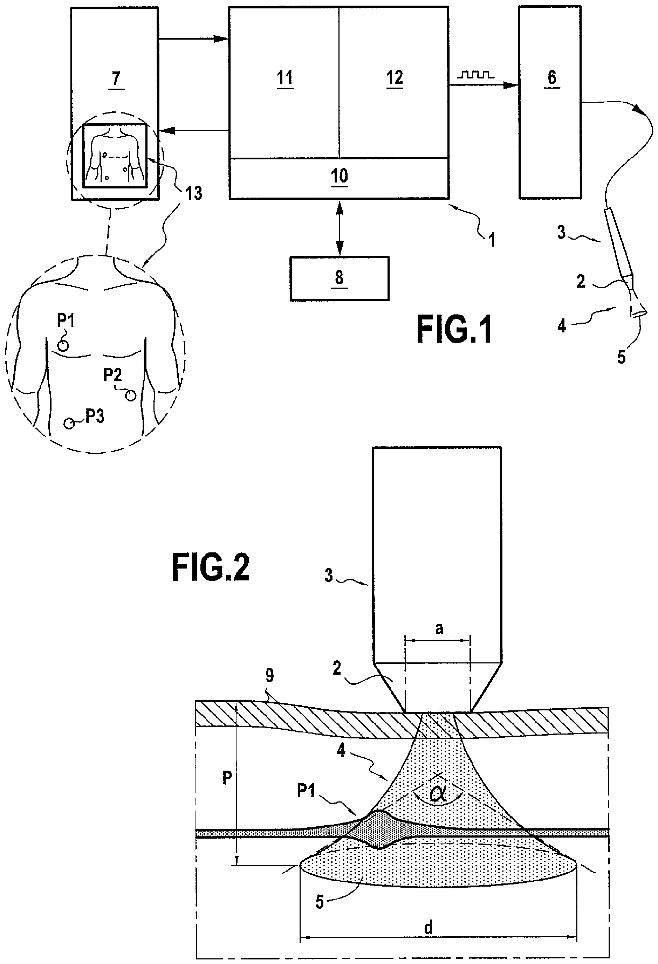

L'appareil de l'invention, tel que représenté schématiquement à la figure 1, comprend une unité de commande 1 qui contrôle une source infrarouge 2 par l'intermédiaire d'une interface de puissance 6 reliée à un moyen d'application 3 d'un faisceau infrarouge 4 émis par la source infrarouge 2.

Une interface utilisateur 7 permet à l'utilisateur de piloter l'unité de commande 1, notamment par exemple pour la mise en route et l'arrêt de l'appareil et des séquences.

Par ailleurs, une unité de stockage 8 est prévue pour stocker notamment des informations qui permettront à l'unité de commande 8 de contrôler l'émission du faisceau infrarouge 4.

La source infrarouge 2 permet donc l'émission d'un faisceau infrarouge 4.

L'appareil est de préférence autonome et dispose alors de sa propre batterie électrique. Alternativement, il peut être relié à une source d'alimentation électrique extérieure.

L'unité de commande 1 comprend notamment trois modules principaux, à savoir un module de calcul 10 par exemple du type microprocesseur 10, un module électronique 11 de gestion des commandes transmises par l'interface utilisateur 7, et un module électronique 12 d'élaboration des impulsions d'excitation de la source infrarouge 2.

L'unité de commande 1 peut par exemple être paramétrée en sorte que, en fonctionnement, l'appareil permette l'émission d'un faisceau infrarouge 4 de longueur d'ondes comprise entre 600 nm et 1000 nm. En outre, la fréquence de découpe des impulsions excitatrices peut par exemple être comprise entre 0 Hz (correspondant à un faisceau continu) et 10 Hz.

La source infrarouge 2 comprend un ou plusieurs émetteurs infrarouges, parmi lesquels au moins une LED infrarouge.

L'unité de commande 1 permet, par l'intermédiaire du module électronique 12 d'élaboration des impulsions excitatrices, de délivrer une ou plusieurs impulsions de forme sensiblement carrée, de durée variable et de fréquence égale à la fréquence de découpe mentionnée plus haut.

Ces impulsions électriques sont délivrées à l'interface de puissance 6. Cette interface de puissance 6 transforme alors ces impulsions en courant d'alimentation de la source infrarouge 2 par une liaison avec le moyen d'application 3, qui est par exemple de type sonde).

Le module de calcul 10 gère la forme des impulsions excitatrices délivrées par le module électronique 12 d'élaboration des impulsions.

Autrement dit, ce module de calcul 10 permet d'élaborer les formes et fréquences des impulsions à émettre, et gère les paramètres de commande.

Parmi les informations stockées par l'unité de stockage 8, on trouve une ou plusieurs séquences de valeurs d'un ou plusieurs paramètres d'application du faisceau, en particulier la durée d'application du faisceau 4 qui correspond à la durée d'émission de ce faisceau 4.

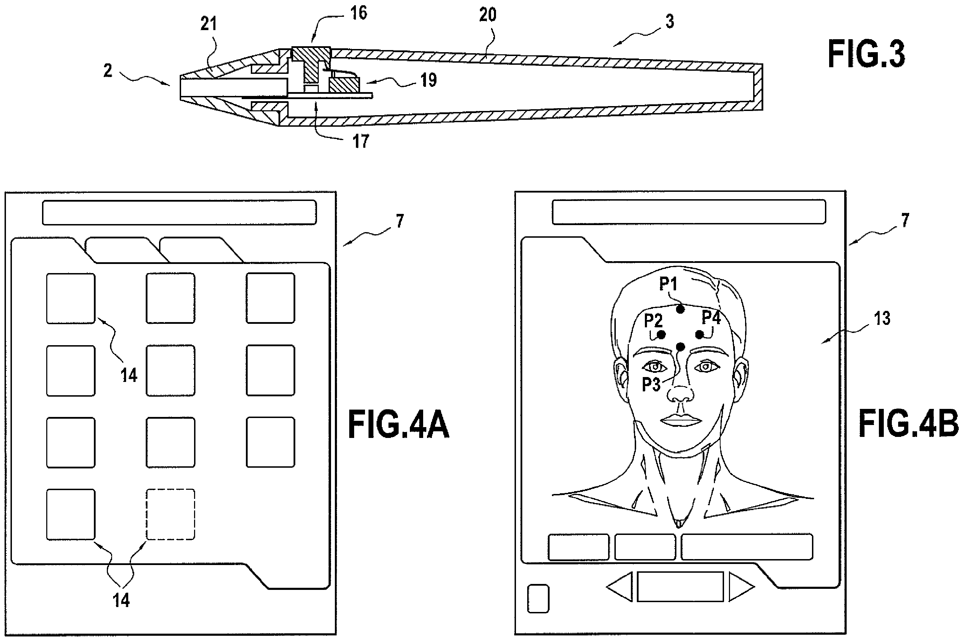

Comme on le verra plus en détail plus loin dans la description, en référence aux figures 4a et 4b, l'interface utilisateur 7 permet de sélectionner une séquence de valeurs du ou des paramètres parmi plusieurs séquences stockées dans l'unité de stockage 8

L'unité de commande 1 est ainsi apte à commander l'émission d'une séquence de faisceaux infrarouge selon la séquence de valeurs sélectionnée.

Par exemple, si le paramètre contrôlé est la durée d'application du faisceau infrarouge 4, et si l'unité de stockage 8 stocke les valeurs successives, en secondes, 20, 40 et 20, l'unité de commande 1 commandera l'émission d'un premier faisceau infrarouge 4 pendant 20 secondes, puis d'un deuxième faisceau infrarouge 4 pendant 40 secondes et enfin d'un troisième faisceau infrarouge 4 pendant de nouveau 20 secondes.

L'utilisateur peut donc appliquer successivement ces trois faisceaux infrarouge 4 sur une séquence de trois points, ou sur deux points, ou encore sur un seul point.

Plus généralement, la séquence de valeurs comprend un nombre de valeurs supérieur à 3, pouvant aller jusqu'à 40, ce qui permet donc d'appliquer successivement des faisceaux infrarouge sur une séquence de 1 à 40 points.

On peut prévoir également que l'unité de stockage 8 stocke les positions respectives des points P₁, P₂, P₃ de la séquence de points sur lesquels les faisceaux infrarouge 4 doivent être appliqués.

Dans ce cas, l'interface utilisateur 7 comprend des moyens 13 d'indication des positions des points P₁, P₂, P₃.

Dans l'exemple de réalisation représenté à la figure 1, ces moyens d'indication 13 prennent la forme d'une représentation graphique 13 de la partie du sujet, sur laquelle sont positionnés les points P₁, P₂, P₃ de la séquence de points sur laquelle la séquence de faisceaux infrarouge 4 doit être appliquée.

Cette représentation graphique, peut prendre la forme d'une image ou d'un dessin affiché sur un écran de l'interface utilisateur 7, tel qu'on le verra plus en détail relativement aux figures 4a et 4b.

Les paramètres d'application du faisceau infrarouge, et d'autres paramètres tels que le nom de la séquence de points, correspondant à un type de soin particulier, des paramètres correspondant au mode de stimulation (par exemple normal ou intensif), peuvent être affichés et/ou modifiés par l'intermédiaire de cette interface 7

D'autres moyens d'indication 13 pourraient être utilisés, tel que des moyens d'indication sonores.

De tels moyens d'indication sonores pourraient aussi permettre de signaler à l'opérateur le début et la fin de l'émission infrarouge.

L'indication de la position des points P₁, P₂, P₃ est ainsi donnée à l'utilisateur, à partir des informations de position stockées dans l'unité de stockage 8.

Tel que représenté plus précisément à la figure 2, en fonctionnement, le faisceau infrarouge 4 émis par la source infrarouge 2, par exemple de type LED infrarouge, associée au moyen d'application 3, est divergent.

Ce faisceau infrarouge 4 présente un angle α d'ouverture de préférence de l'ordre de 100 ° pour un diamètre a, à la sortie de la source lumineuse 2, de l'ordre de 6 à 7 mm.

La zone 5, sensiblement circulaire, ainsi stimulée par l'application du faisceau infrarouge 4 se trouve à une profondeur p sous la surface 9 du sujet, par exemple la peau dans le cas de l'acupuncture, de l'auriculothérapie, ou de la réflexothérapie, qui est inférieure à 6 cm.

A cette profondeur, le diamètre d de cette zone 5 circulaire est compris entre 0.7 cm et 3 cm, lorsque le moyen d'application 3 se trouve en contact direct avec la surface 9 du sujet.

Ceci permet de stimuler de façon appropriée le point P₁ situé sous la surface, tel qu'un point d'acupuncture, d'auriculothérapie, ou de réflexothérapie, situé sous la peau d'un sujet, selon les principes régissant l'acupuncture ou l'auriculothérapie mentionnés en introduction de la présente description.

On a représenté par ailleurs à la figure 3 un exemple de réalisation du moyen d'application 3 permettant d'appliquer le faisceau infrarouge.

Dans cet exemple, le moyen d'application 3 prend une forme longiligne de type stylet, comprenant un corps principal creux 20, fermé en l'une de ses extrémités par un embout 21 dans lequel est logée une LED 2.

On pourrait envisager d'utiliser plusieurs émetteurs infrarouge 2, dont au moins une LED infrarouge.

Cette LED 2 est reliée à un circuit électrique connecté à l'appareil de l'invention représenté à la figure 1, et est activée par un moyen 16 de déclenchement manuel de l'émission d'un faisceau infrarouge.

Dans cet exemple, le moyen de déclenchement manuel 16 est disposé directement sur le moyen d'application 3, précisément sur le corps 20 de ce moyen d'application 3.

Ce moyen de déclenchement manuel 16 prend la forme d'un bouton qui vient faire basculer un switch 19 pour réaliser l'activation.

Dans cet exemple, le moyen d'application 3 comprend également une ou plusieurs LED 17, constituant des témoins lumineux 17 d'activation de l'émission du faisceau infrarouge par la source infrarouge 2.

Ces témoins lumineux 17 sont reliés au même circuit électrique que celui auquel est reliée la source infrarouge 2.

Dans cet exemple, le ou les témoins lumineux 17 sont situés à l'intérieur du corps 20 du moyen d'application 3, en regard du bouton de déclenchement manuel 16. Ce bouton 16 est translucide, de sorte que, lors de l'activation de la source infrarouge 2, le ou les témoins lumineux 17 sont également activés et émettent une lumière visible par l'utilisateur à travers le bouton 16.

L'interface utilisateur 7 de l'appareil selon l'invention est représenté dans un exemple de réalisation aux figures 4a et 4b, selon deux états respectifs.

Cette interface utilisateur 7 comprend classiquement un périphérique d'entrée et un périphérique de sortie.

Il peut s'agir de boutons, par exemple de type boutons poussoirs de commande, permettant notamment de sélectionner les différents modes d'application mentionnés plus haut, ainsi que des leds témoins de fonctionnement, le tout disposé en façade de l'appareil.

Il peut s'agir également d'un écran et d'un clavier, disposés en façade de l'appareil.

Dans l'exemple représenté aux figures 4a et 4b, l'interface utilisateur 7 prend la forme d'un écran tactile 7.

L'écran tactile comprend des moyens 14 de sélection de la séquence de valeurs du ou des paramètres d'application du faisceau, tel que la durée d'application, éventuellement associées à des positions de points P₁, P₂, P₃ tels que présentés plus haut.

Ces moyens de sélection 14, ou premiers moyens de sélection 14, prennent la forme de boutons 14, correspondant à des séquences d'application de faisceau lumineux enregistrées dans l'unité de stockage 8.

En appuyant sur un des boutons 14, l'utilisateur sélectionne donc une séquence de faisceaux infrarouge paramétrés à appliquer par l'intermédiaire du moyen d'application 3.

En sélectionnant une des séquence enregistrées dans l'unité de stockage 8, et dans le cas où il est prévu dans l'appareil un moyen 13 d'indication des points P₁, P₂, P₃, P₄ de la séquence de points sur laquelle la séquence de faisceaux infrarouge doit être appliquée, sous la forme d'une représentation graphique 13, l'interface utilisateur 7 affiche cette représentation graphique 13 avec les points P₁, P₂, P₃, P₄ positionnés.

C'est ce qui est représenté à la figure 4b.

Ainsi, l'utilisateur se voit guidé dans l'application des faisceaux infrarouge, non seulement en termes de durée d'application, mais également en termes de position des points d'application.

Il est possible de prévoir d'afficher tous les points P₁, P₂, P₃, P₄ de la séquence, et de les numéroter ou de les nommer.

On peut également prévoir de les afficher un par un, en sorte également de guider l'utilisateur dans l'ordre dans lequel les faisceaux infrarouge doivent être appliqués. Tous les points P₁, P₂, P₃, P₄ sont affichés avec des indicateurs différents, par exemple une couleur différente et/ou un clignotement, selon que le point est « traité » (faisceau infrarouge déjà appliqué), « en cours » (faisceau infrarouge en cours d'application), ou « à traiter » (faisceau infrarouge à appliquer).

L'interface utilisateur 7 peut aussi comprendre des moyens de sélection, ou deuxièmes moyens de sélection, permettant de sélectionner un mode d'application d'une séquence de faisceaux infrarouge 4, parmi un certain nombre de modes possibles, dont au moins un mode manuel et/ou un mode automatique.

Dans le mode d'application manuel, l'émission de chaque faisceau infrarouge 4 de la séquence est déclenchée manuellement par l'utilisateur, par le moyen de déclenchement 13 prévu à cet effet par exemple dans l'interface utilisateur 7 ou sur l'appareil, ou sur le moyen d'application 3 tel que décrit plus haut en référence à la figure 3.

Dans le mode d'application automatique, l'utilisateur déclenche manuellement l'émission du premier faisceau infrarouge 4 de la séquence, par le moyen de déclenchement 13 prévu à cet effet par exemple dans l'interface utilisateur 7 ou sur l'appareil, ou sur le moyen d'application 3 tel que décrit plus haut en référence à la figure 3.

Ensuite, les faisceaux en référence à la figure 3 4 suivants dans la séquence sont déclenchés automatiquement par l'unité de commande 1, avec un laps de temps prédéterminé entre la fin d'émission d'un faisceau infrarouge 4 et le début de l'émission du faisceau infrarouge 4 suivant dans la séquence.

On peut prévoir que ce laps de temps prédéterminé entre la fin d'émission d'un faisceau infrarouge 4 et le début de l'émission du faisceau infrarouge 4 suivant dans la séquence, soit constant sur toute la séquence.

On peut également prévoir que ce laps de temps prédéterminé entre la fin d'émission d'un faisceau infrarouge 4 et le début de l'émission du faisceau infrarouge 4 suivant dans la séquence, varie pendant la séquence.

Le ou les laps de temps prédéterminés peuvent être stockés dans l'unité de stockage 8.

On peut prévoir également, dans l'interface utilisateur 7 des moyens de sélection, ou troisièmes moyens de sélection, permettant à l'utilisateur de sélectionner un mode dit « expert ».

Dans ce mode expert, l'utilisateur peut, par l'intermédiaire de l'interface utilisateur 7, saisir et enregistrer dans l'unité de stockage 8 une séquence de valeurs du ou des paramètres de faisceau infrarouge gérés, dont la durée d'application, et/ou, éventuellement, une séquence de position de points correspondante.

L'interface utilisateur 7 permet également de sélectionner un mode d'application par impulsion, et un mode d'application continu, par l'intermédiaire de quatrièmes moyens de sélection.

Dans le mode d'application par impulsion, et en référence à la figure 1, le module électronique 11 de gestion des commandes de l'unité de commande 1 donne instruction au module électronique 12 d'élaboration des impulsions excitatrices, de délivrer à l'interface de puissance 6 un train d'impulsions pendant un temps déterminé pour générer un faisceau infrarouge 4.

Dans le mode d'application continu, le module électronique 11 de gestion des commandes de l'unité de commande 1 donne instruction au module électronique 12 d'élaboration des impulsions, de délivrer à l'interface de puissance 6 une seule impulsion de durée déterminée pour générer le faisceau infrarouge 4.

L'interface utilisateur 7 permet également de régler le temps d'application du train d'impulsions dans le mode continu, et la durée de l'impulsion unique dans le mode discontinu.

Néanmoins, ce temps d'application peut être préréglé, de préférence de façon non modifiable, par exemple à une valeur de 20 secondes.

Le procédé consiste donc en l'application d'une stimulation locale à un sujet au moyen de l'appareil présenté ci-dessus.

Ainsi, le moyen 3 d'application, contrôlé par l'unité de commande 1, permet une application directement au contact avec la surface 9 du sujet, du faisceau infrarouge 4 émis par la source infrarouge 2.

L'utilisateur contrôle donc l'unité de commande 1 par l'intermédiaire de l'interface utilisateur 7, en sorte que cette unité de commande 1 délivre une ou plusieurs impulsions, de préférence carrées ou sinusoïdales, de durée variable.

Comme indiqué plus haut, cette ou ces impulsions sont délivrées à une interface de puissance 6 qui transforme les impulsions en courant d'alimentation de la source lumineuse 2.

L'utilisateur, par l'intermédiaire de l'interface utilisateur 7, a le choix entre un mode d'application par impulsion ou continu, dans lesquels l'unité de commande 1 délivre respectivement un train d'impulsions pendant un temps déterminé et une seule impulsion de durée déterminée.

L'utilisateur, par l'intermédiaire de l'interface utilisateur 7, a également le choix entre un mode d'application manuel ou automatique, déjà décrits plus haut.

Egalement comme indiqué plus haut, l'utilisateur, par l'intermédiaire de l'interface utilisateur 7, commande à l'unité de commande 1 le déclenchement de l'émission d'une séquence de faisceaux infrarouge 4 dont un ou plusieurs paramètres, notamment la durée d'émission, sont contrôlés par cette unité de commande 1, grâce à une séquence de valeurs de paramètres stockée dans l'unité de stockage 8.

Les valeurs des paramètres peuvent être importées dans et exportées de l'unité de stockage 8, par l'intermédiaire d'une connexion adaptée.

Par exemple, l'appareil peut être relié à un ordinateur par une liaison filaire, par exemple de type USB, en sorte de télécharger dans l'unité de stockage 8 des séquences de valeurs de paramètres correspondant à des protocoles donnés.

A l'inverse, il est possible de télécharger de telles séquences de valeurs de paramètres depuis l'unité de stockage 8 vers un ordinateur.

La liaison peut éventuellement se faire d'un appareil selon l'invention à un autre appareil selon l'invention, sans passer par un ordinateur.

La liaison peut aussi être non filaire, par exemple de type WiFi ou Bluetooth.

Il est également possible de prévoir une unité de stockage 8 amovible dans l'appareil, par exemple de type carte mémoire ou clé USB, qui peut ensuite être lu par un ordinateur classique.

Comme indiqué plus haut, les valeurs des paramètres, ainsi, éventuellement, que les positions des points correspondants, peuvent aussi être saisies manuellement par l'utilisateur au moyen de l'interface utilisateur 7, puis enregistrées dans l'unité de stockage 8, lui permettant ainsi de définir ses propres programmes ou séquences d'application de faisceaux infrarouge.

Les différents moyens de sélection présentés dans la présente description, et appelés premiers, deuxièmes, troisièmes et quatrièmes moyens de sélection, peuvent classiquement consister en des boutons de sélection affichés sur l'écran tactile constituant l'interface utilisateur 7.

En dehors des fonctions de réglage classique de l'interface utilisateur, telles que le choix de la langue, le réglage du volume sonore pour la diffusion d'informations sonores, le réglage du contraste de l'écran, on peut prévoir des fonctions de réglage propre à l'utilisation en acupuncture, auriculothérapie ou réflexothérapie, ainsi qu'un compteur de séquences permettant de connaître le taux d'utilisation de l'appareil.

On peut prévoir également des moyens de navigation classique dans les différents écrans de l'interface utilisateur.

Enfin, on peut aussi prévoir un moyen, par exemple un bouton, permettant de mettre l'appareil en pause, c'est-à-dire d'interrompre l'application de la séquence de faisceaux infrarouge, de revenir en arrière dans la séquence, c'est-à-dire de revenir à un des faisceaux précédents de la séquence, ou d'avancer dans la séquence, c'est-à-dire de passer directement à un des faisceaux suivants dans la séquence.

Il est rappelé enfin que l'ensemble de la présente description est donnée à titre d'exemple et n'est pas limitatif de l'invention.

## Revendications

1. Appareil destiné à appliquer une stimulation locale sur un point (P₁, P₂, P₃) d'un sujet, notamment un point (P₁, P₂, P₃) d'acupuncture, d'auriculothérapie ou de réflexothérapie, dit point réflexe, ou une terminaison nerveuse, comprenant une unité de commande (1) contrôlant une source infrarouge (2) pour l'émission d'un faisceau infrarouge (4), une unité de stockage (8), un moyen (3) d'application dudit faisceau infrarouge (4) sur ledit point (P₁, P₂, P₃), et une interface utilisateur (7), l'unité de stockage (8) étant configurée pour stocker au moins une séquence de valeurs d'un ou plusieurs paramètres d'application du faisceau (4), notamment la durée et/ou la puissance optique, l'interface utilisateur (7) comprenant des premiers moyens de sélection (14) de ladite séquence de valeurs, et l'unité de commande (1) étant configurée pour contrôler le ou lesdits paramètres et pour commander l'émission de plusieurs faisceaux infrarouge (4) successifs selon ladite séquence de valeurs, en sorte de permettre l'application sur une séquence de points (P₁, P₂, P₃), par l'intermédiaire du moyen d'application (3), d'une séquence de faisceaux infrarouge (4) correspondant à ladite séquence de valeurs, ledit appareil étant **caractérisé en ce que** l'unité de stockage est configurée pour stocker les positions respectives des points (P₁, P₂, P₃) de la séquence sur lesquels les faisceaux infrarouge (4) successifs doivent être respectivement appliqués, et **en ce que** l'interface utilisateur (7) comprend des moyens (13) d'indication desdites positions prenant la forme d'une représentation graphique affichée sur un écran d'au moins une partie du sujet sur laquelle sont positionnés les points (P₁, P₂, P₃) de la séquence, l'interface utilisateur étant configurée pour afficher des indicateurs différents pour chacune desdites positions, selon que le point est « traité », « en cours » ou « à traiter ».

2. Appareil selon la revendication **1, caractérisé en ce que** l'interface utilisateur (7) comprend des deuxièmes moyens de sélection d'au moins un mode d'application manuel dans lequel l'émission de chaque faisceau infrarouge (4) de la séquence de faisceaux infrarouge (4) est déclenchée manuellement, et/ou d'au moins un mode d'application automatique dans lequel l'émission du premier faisceau infrarouge (4) de la séquence de faisceaux infrarouge (4) est déclenchée manuellement et l'émission de chaque faisceau infrarouge (4) suivant dans la séquence de faisceaux infrarouge (4) est déclenchée automatiquement après un laps de temps prédéterminé à partir de la fin de l'émission du précédent faisceau infrarouge (4) dans ladite séquence.

3. Appareil selon la revendication **2, caractérisé en ce que** le laps de temps prédéterminé est constant sur toute la séquence de faisceau infrarouge (4), de préférence stocké dans l'unité de stockage (8).

4. Appareil selon la revendication **2, caractérisé en ce que** le laps de temps prédéterminé varie sur la séquence de faisceau infrarouge (4), les différents laps de temps prédéterminés étant de préférence stockés dans l'unité de stockage (8).

5. Appareil selon l'une quelconque des revendications **1 à 4, caractérisé en ce qu'**il comprend des troisièmes moyens de sélection permettant de sélectionner au moins un mode expert dans lequel l'interface utilisateur (7) permet la saisie et l'enregistrement dans l'unité de stockage (8) des positions des points d'une séquence de points, et/ou la saisie et l'enregistrement dans ladite unité de stockage (8) d'une séquence correspondante de valeurs du ou des paramètres d'application du faisceau infrarouge (4).

6. Appareil selon l'une quelconque des revendications **1 à 5, caractérisé en ce qu'**il comprend un moyen (16) de déclenchement manuel de l'émission d'un faisceau infrarouge (4).

7. Appareil selon la revendication **6, caractérisé en ce que** le moyen (16) de déclenchement manuel, par exemple de type bouton de commande (16), est disposé sur le moyen d'application (3).

8. Appareil selon la revendication **7, caractérisé en ce qu'**il comprend au moins un témoin lumineux (17) de fonctionnement du moyen d'application (3), ledit témoin lumineux (17) étant activé par activation du moyen de déclenchement (16).

9. Appareil selon la revendication **8, caractérisé en ce que** le moyen de déclenchement (16) est translucide et **en ce que** le témoin lumineux (17) est une source de lumière, par exemple de type LED, disposée à l'intérieur du moyen d'application (3) en regard du moyen de déclenchement (16).

10. Appareil selon l'une quelconque des revendications **1 à 9, caractérisé en ce que** l'interface utilisateur (7) comprend des quatrièmes moyens de sélection d'au moins un mode d'application par impulsion dans lequel l'unité de commande (1) permet l'émission d'un faisceau lumineux (4) en délivrant pendant un temps déterminé un train d'impulsions, et/ou au moins un mode d'application continu dans lequel l'unité de commande (1) permet l'émission d'un faisceau infrarouge (4) en délivrant une seule impulsion de durée déterminée.

11. Appareil selon la revendication **10, caractérisé en ce que** l'interface utilisateur (7) permet de régler le temps d'application du train d'impulsions dans le mode par impulsion, et/ou la durée de l'impulsion dans le mode continu.

12. Appareil selon l'une quelconque des revendications **1 à 11, caractérisé en ce qu'**il permet, en fonctionnement, l'émission d'un faisceau infrarouge (4) divergeant et présentant un angle (a) d'ouverture sensiblement égal à 100°, de sorte que lorsque le moyen (3) d'application se trouve en contact direct avec la surface (9) du sujet, la zone (5) stimulée par application dudit faisceau infrarouge (4), à une profondeur (p) sous la surface (9) dudit sujet sensiblement comprise entre 0 cm et 6 cm, est sensiblement circulaire et présente un diamètre (d) sensiblement compris entre 0,7 cm et 3 cm.

13. Appareil selon l'une quelconque des revendications **1 à 12, caractérisé en ce qu'**il permet l'émission d'un faisceau infrarouge (4) de longueur d'ondes comprise entre 600 nm et 1000 nm.

14. Appareil selon l'une quelconque des revendications **1 à 13, caractérisé en ce que** la source infrarouge (2) comprend plusieurs émetteurs infrarouge.

15. Appareil selon l'une quelconque des revendications **1 à 14, caractérisé en ce que** l'unité de commande (1) permet de délivrer une ou plusieurs impulsions de forme sensiblement carrée, de durée variable, et de fréquence égale à la fréquence de découpe, à une interface de puissance (6) qui transforme lesdites impulsions en courant d'alimentation de la source infrarouge (2).

16. Appareil selon la revendication **15, caractérisé en ce que** la fréquence de découpe des impulsions est comprise entre 0 Hz et 10 Hz.

## Patentansprüche

1. Vorrichtung zur lokalen Stimulation eines Punktes (P₁, P₂, P₃) einer Person, insbesondere eines Akupunktur-, Aurikulotherapie- oder Reflextherapiepunktes (P₁, P₂, P₃), Reflexpunkt genannt, oder eines Nervenabschlusses, umfassend eine Steuereinheit (1), die eine Infrarotquelle (2) für die Emission eines Infrarotstrahls (4) steuert, eine Speichereinheit (8), ein Mittel (3) zum Anwenden des Infrarotstrahls (4) auf dem Punkt (P₁, P₂, P₃) und eine Benutzerschnittstelle (7), wobei die Speichereinheit (8) dazu ausgelegt ist, mindestens eine Folge von Werten eines oder mehrerer Parameter der Anwendung des Strahls (4) zu speichern, insbesondere die Dauer und/oder die optische Leistung, die Benutzerschnittstelle (7) erste Auswahlmittel (14) der Folge von Werten umfasst und die Steuereinheit (1) dazu ausgelegt ist, den oder die Parameter zu steuern und die Emission mehrerer aufeinanderfolgender Infrarotstrahlen (4) entsprechend der Folge von Werten so zu steuern, dass eine der Folge von Werten entsprechende Folge von Infrarotstrahlen (4) mit Hilfe des Anwendungsmittels (3) auf einer Folge von Punkten (P₁, P₂, P₃) angewendet werden kann, wobei die Vorrichtung **dadurch gekennzeichnet, dass** die Speichereinheit dazu ausgelegt ist, die jeweiligen Positionen der Punkte (P₁, P₂, P₃) der Folge, an denen aufeinanderfolgende Infrarotstrahlen (4) jeweils angewendet werden sollen, zu speichern, und dass die Benutzerschnittstelle (7) Mittel (13) zum Anzeigen der Positionen in Form einer graphischen Darstellung aufweist, die auf einem Bildschirm zumindest bezüglich des Teils der Person angezeigt wird, in dem die Punkte (P₁, P₂, P₃) der Folge positioniert sind, wobei die Benutzerschnittstelle dazu ausgelegt ist, verschiedene Indikatoren für jede dieser Positionen anzuzeigen, je nachdem, ob der Punkt "bearbeitet", "in Bearbeitung" oder "zu bearbeiten" ist.

2. Vorrichtung gemäß Anspruch 1, **dadurch gekennzeichnet, dass** die Benutzerschnittstelle (7) zweite Mittel zur Auswahl mindestens eines manuellen Anwendungsmodus, in dem die Emission jedes Infrarotstrahls (4) der Folge von Infrarotstrahlen (4) manuell ausgelöst wird, und/oder mindestens eines automatischen Anwendungsmodus, in dem die Emission des ersten Infrarotstrahls (4) der Folge von Infrarotstrahlen (4) manuell ausgelöst wird und die Emission jedes nachfolgenden Infrarotstrahls (4) der Folge von Infrarotstrahlen (4) automatisch nach einer vorbestimmten Zeitspanne ab dem Ende der Emission des vorherigen Infrarotstrahls (4) der Folge ausgelöst wird, umfasst.

3. Vorrichtung gemäß Anspruch 2, **dadurch gekennzeichnet, dass** die vorbestimmte Zeitspanne über die gesamte Folge von Infrarotstrahlen (4) konstant ist und vorzugsweise in der Speichereinheit (8) gespeichert wird.

4. Vorrichtung gemäß Anspruch 2, **dadurch gekennzeichnet, dass** die vorbestimmte Zeitspanne über die Folge von Infrarotstrahlen (4) hinweg variiert, wobei die verschiedenen vorbestimmten Zeitspannen vorzugsweise in der Speichereinheit (8) gespeichert werden.

5. Vorrichtung gemäß irgendeinem der Ansprüche 1 bis 4, **dadurch gekennzeichnet, dass** sie dritte Mittel zur Auswahl umfasst, die es ermöglichen, mindestens einen Expertenmodus auszuwählen, in dem die Benutzerschnittstelle (7) das Erfassen und Aufzeichnen der Positionen der Punkte einer Folge von Punkten in der Speichereinheit (8) und/oder das Erfassen und Aufzeichnen einer entsprechenden Folge von Werten des oder der Anwendungsparameter(s) des Infrarotstrahls (4) in der Speichereinheit (8) ermöglicht.

6. Vorrichtung gemäß einem der Ansprüche 1 bis 5, **dadurch gekennzeichnet, dass** sie ein Mittel (16) zum manuellen Auslösen der Emission eines Infrarotstrahls (4) umfasst.

7. Vorrichtung gemäß Anspruch 6, **dadurch gekennzeichnet, dass** das Mittel (16) zum manuellen Auslösen, z. B. vom Typ Betätigungsknopf (16), auf dem Anwendungsmittel (3) angeordnet ist.

8. Vorrichtung gemäß Anspruch 7, **dadurch gekennzeichnet, dass** sie mindestens eine Leuchtanzeige (17) des Betriebs des Anwendungsmittels (3) umfasst, wobei die Leuchtanzeige (17) durch Aktivierung des Auslösemittels (16) aktiviert wird.

9. Vorrichtung gemäß Anspruch 8, **dadurch gekennzeichnet, dass** das Auslösemittel (16) lichtdurchlässig ist und dass es sich bei der Leuchtanzeige (17) um eine Lichtquelle, z. B. des Typs LED, handelt, die im Inneren des Anwendungsmittels (3) gegenüber dem Auslösemittel (16) angeordnet ist.

10. Vorrichtung gemäß einem der Ansprüche 1 bis 9, **dadurch gekennzeichnet, dass** die Benutzerschnittstelle (7) vierte Mittel zur Auswahl mindestens eines Impulsanwendungsmodus, in dem die Steuereinheit (1) die Emission eines Lichtstrahls (4) durch Abgabe einer Impulsfolge für eine bestimmte Zeit ermöglicht, und/oder mindestens eines kontinuierlichen Anwendungsmodus, in dem die Steuereinheit (1) die Emission eines Infrarotstrahls (4) durch Senden eines einzelnen Impulses mit festgelegter Dauer ermöglicht, umfasst.

11. Vorrichtung gemäß Anspruch 10, **dadurch gekennzeichnet, dass** die Benutzerschnittstelle (7) es ermöglicht, die Anwendungszeit der Impulsfolge im Impulsmodus und/oder die Impulsdauer im kontinuierlichen Modus einzustellen.

12. Vorrichtung gemäß einem der Ansprüche 1 bis 11, **dadurch gekennzeichnet, dass** sie während des Betriebs die Emission eines divergierenden Infrarotstrahls (4) mit einem Öffnungswinkel (a) von im Wesentlichen 100° ermöglicht, sodass, wenn das Anwendungsmittel (3) in direktem Kontakt mit der Oberfläche (9) der Person steht, der Bereich (5), der durch Anwendung des Infrarotstrahls (4) stimuliert wird, in einer Tiefe (p) unterhalb der Oberfläche (9) der Person von ungefähr 0 cm bis 6 cm, im Wesentlichen kreisförmig ist und einen Durchmesser (d) von ungefähr 0,7 cm bis 3 cm aufweist.

13. Vorrichtung gemäß einem der Ansprüche 1 bis 12, **dadurch gekennzeichnet, dass** sie die Emission eines Infrarotstrahls (4) mit einer Wellenlänge zwischen 600 nm und 1000 nm ermöglicht.

14. Vorrichtung gemäß einem der Ansprüche 1 bis 13, **dadurch gekennzeichnet, dass** die Infrarotquelle (2) eine Vielzahl von Infrarot-Strahlern umfasst.

15. Vorrichtung gemäß einem der Ansprüche 1 bis 14, **dadurch gekennzeichnet, dass** die Steuereinheit (1) es ermöglicht, einen oder mehrere Impulse von im Wesentlichen quadratischer Form, variabler Dauer und einer Frequenz, die der Schnittfrequenz entspricht, an eine Leistungsschnittstelle (6) zu senden, die diese Impulse in einen Versorgungsstrom der Infrarotquelle (2) umwandelt.

16. Vorrichtung gemäß Anspruch 15, **dadurch gekennzeichnet, dass** die Schnittfrequenz der Impulse zwischen 0 Hz und 10 Hz liegt.

## Claims

1. An apparatus for locally stimulating a point (P₁, P₂, P₃) of a subject, in particular in acupuncture, auriculotherapy or reflex therapy, called trigger area, or a nerve ending, comprising a control unit (1) controlling an infrared source (2) for emitting an infrared beam (4), a storage unit (8), a means (3) for applying said infrared beam (4) on said point (P₁, P₂, P₃), and a user interface (7), the storage unit (8) being configured to store at least one sequence of values of one or several application parameters of the beam (4), in particular the duration and/or the optical power, the user interface (7) comprising first selection means (14) for selecting said sequence of values, and the control unit (1) being configured to control said parameter(s) and to command the emission of several successive infrared beams (4) according to said sequence of values, so as to allow the application on a sequence of points (P₁, P₂, P₃), via the application means (3), of a sequence of infrared beams (4) corresponding to said sequence of values, said apparatus being **characterized in that** the storage unit is configured to store the respective positions of the points (P₁, P₂, P₃) of the sequence in which the successive infrared beams (4) must respectively be applied, and **in that** the user interface (7) comprises means (13) for indicating said positions assuming the form of a graphic representation displayed on a screen of at least part of the subject on which the points (P₁, P₂, P₃) of the sequence are positioned, the user interface being configured to display different indicators for each of said positions, depending on whether the point is "processed", "in progress" or "to be processed".

2. The apparatus according to claim **1, characterized in that** the user interface (7) comprises second means for selecting at least one manual application mode in which the emission of each infrared beam (4) of the sequence of infrared beams (4) is triggered manually, and/or at least one automatic application mode in which the emission of the first infrared beam (4) of the sequence of infrared beams (4) is triggered manually and the emission of each following infrared beam (4) in the sequence of infrared beams (4) is triggered automatically after a predetermined length of time from the end of the emission of the previous infrared beam (4) in said sequence.

3. The apparatus according to claim **2, characterized in that** the predetermined length of time is constant over the entire infrared beam sequence (4), preferably stored in the storage unit (8).

4. The apparatus according to claim **2, characterized in that** the predetermined length of time varies over the infrared beam (4) sequence, the various predetermined lengths of time preferably being stored in the storage unit (8).

5. The apparatus according to any one of claims **1 to 4, characterized in that** it comprises third selection means making it possible to select at least an expert mode in which the user interface (7) allows the entry and recording in the storage unit (8) of the positions of the points of a sequence of points, and/or the entry and recording in said storage unit (8) of a corresponding sequence of values of the application parameter(s) of the infrared beam (4).

6. The apparatus according to any one of claims **1 to 5, characterized in that** it comprises a means (16) for manual triggering of the emission of an infrared beam (4).

7. The apparatus according to claim **6, characterized in that** the manual triggering means (16), for example of the control button type (16), is positioned on the application means (3).

8. The apparatus according to claim **7, characterized in that** it comprises at least one lighted indicator (17) for the operation of the application means (3), said lighted indicator (17) being activated by activating the triggering means (16).

9. The apparatus according to claim **8, characterized in that** the triggering means (16) is translucent and **in that** the lighted indicator (17) is a light source, for example of the LED type, positioned inside the application means (3) across from the triggering means (16).

10. The apparatus according to any one of claims **1 to 9, characterized in that** the user interface (7) comprises fourth means for selecting at least a pulsed application mode in which the control unit (1) allows the emission of a light beam (4) by delivering, for a determined length of time, a series of pulses, and/or at least one continuous application mode in which the control unit (1) allows the emission of an infrared beam (4) by delivering a single pulse with a determined duration.

11. The apparatus according to claim **10, characterized in that** the user interface (7) makes it possible to adjust the application time of the series of pulses in the pulsed mode, and/or the duration of the pulse in the continuous mode.

12. The apparatus according to any one of claims **1 to 11, characterized in that** it allows, during operation, the emission of an infrared beam (4) diverging and having an opening angle (a) substantially equal to 100°, such that when the application means (3) is in direct contact with the surface (9) of the subject, the area (5) stimulated by application of said infrared beam (4), at a depth (p) below the surface (9) of said subject substantially comprised between 0 cm and 6 cm, is substantially circular and has a diameter (d) substantially comprised between 0.7 cm and 3 cm.

13. The apparatus according to any one of claims **1 to 12, characterized in that** it allows the emission of an infrared beam (4) with wavelengths comprised between 600 nm and 1000 nm.

14. The apparatus according to any one of claims **1 to 13, characterized in that** the infrared source (2) comprises several infrared emitters.

15. The apparatus according to any one of claims **1 to 14, characterized in that** the control unit (1) makes it possible to deliver one or several substantially square pulses, with a variable duration, and a frequency equal to the quench frequency, to a power interface (6) that converts said pulses into supply current of the infrared source (2).

16. The apparatus according to claim **15, characterized in that** the quench frequency of the pulses is comprised between 0 Hz and 10 Hz.
